# EUROPEAN PATENT SPECIFICATION

(11) **EP 1 278 521 B1**
(45) Date of publication and mention of the grant of the patent: **30.11.2005**
(21) Application number: 01927530.4
(22) Date of filing: 27.04.2001
(51) Int. Cl.: A61K 31/505, A61K 9/127, A61P 35/00, A61P 35/02

(54) **N-HETEROCYCLIC SUBSTITUTED SALICYLATES FOR THE TREATMENT OF CANCER**
N-HETEROSUBSTITUIERTEN SALICYLATEN ZUR BEHANDLUNG VON KREBS
DES SALICYLATES N-HETEROCYCLIQUES SUBSTITUES DESTINES AU TRAITEMENT DU CANCER

(30) Priority: 28.04.2000 CA 2307278; 28.04.2000 US 200358 P
(43) Date of publication of application: 29.01.2003
(73) Proprietor: THE UNIVERSITY OF BRITISH COLUMBIA, Vancouver, British Columbia V6T 1Z3 (CA)
(72) Inventor: GOUT, Peter, Wilhelm, Vancouver, British Columbia V5Z 1L3 (CA); BRUCHOVSKY, Nicholas, Vancouver, British Columbia V5Z 1L3 (CA)
(74) Representative: Atkinson, Jonathan David Mark
(86) International application number: PCT/CA2001/000570
(87) International publication number: WO 2001/082907

(56) References cited:
- EP-A- 0 513 434
- US-A- 2 396 145
- US-A- 5 556 855
- NARANG, V.S.; GOUT, P.W.; BUCKLEY, D.J.; BUCKLEY, A.R.: "Antagonism of the xc- cystine/ glutamate antiporter by sulfasalazine inhibits proliferation of human carcinoma cells" PROC. AM. ASSOC. CANCER RES., vol. 42, March 2001 (2001-03), page 788 XP001026283

## Description

This invention relates to the use of N-heterocyclic substituted salicylabes for the preparation of medicaments for use in the treatment of cancer.

### Background

Cystine, or its reduced form, cysteine, is an essential amino acid for a variety of cancers, including those derived from myeloid and lymphoid tissues as well as lymphoblastic, lymphocytic and myelocytic leukemias. Cystine and cysteine are referred to collectively herein by the term "cyst(e)ine".

While lymphoid cells can readily take up cysteine from their extracellular environment, they have in general a low uptake capability for cystine, the predominant form of the amino acid in the blood and culture medium. *In vitro,* the cyst(e)ine requirement of lymphoid cells can be accommodated by the presence in the medium of cystine at elevated concentrations or, by the presence of a cystine uptake-enhancing thiol such as 2-mercaptoethanol (2-ME). *In vivo,* lymphoid cell proliferation apparently depends significantly on the supply of cysteine by neighbouring body cells such as macrophages and fibroblasts which take up cystine from circulation by a cystine/glutamate antiporter plasma membrane transport system (designated x_{c}⁻) and release cysteine into the extracellular space. Studies have shown that malignant progression of Nb2 lymphoma cells is associated with an increased ability to take up extracellular cystine and inhibition of the x_{c}⁻ system abrogates proliferation of lymphoma cell lines in the absence of extrinsic cysteine or 2-ME. Thus, it was suggested that the x_{c}⁻ system may provide a target for T cell cancer therapy (Gout, *P.W. et al.* (1997) Leukemia 11:1329-1337).

The x_{c}⁻ transport system can be inhibited by monosodium glutamate and there is some suggestion in the literature that the transport system may also be inhibited by non-steroidal anti-inflammatory drugs (NSAIDs), including salicylates. (See: S. Bannai and H. Kasuga (1985) Biochem. Pharmacol. 34:1852.) It has also been indicated in various studies that certain salicylates (notably, acetylsalicylic acid and its metabolite, salicylate) inhibit proliferation or induce apoptosis in some cancer cell lines, including colorectal, breast, pancreatic and lymphocytic leukemia. The latter salicylates are NSAIDs and various mechanisms have been proposed for their action, including enhancing tumor necrosis factor activation by inhibiting nuclear factor kappa B-dependent anti-apoptotic genes. However, studies involving 5' substituted salicylates, including 5' aminosalicylic acid (5-ASA) and sulfasalazine, do not show an anti-cancer effect of such compounds (see: Ritland, S.R. *et al.* (1999) Clin. Cancer Res. 5:855-63). Furthermore, the literature relating to cancer treatment which mention sulfasalazine do not describe any useful effect of sulfasalazine in cancer treatment when applied as a single drug.

United States Patent No. 5,670,502 (Brown) describes a method of treating solid cancer tumors containing hypoxic cells with a combination of a benzotriazine chemotherapy agent followed by a further agent to which the tumor is susceptible. Preferably, the further agent is one that acts on normally oxygenated cancer cells. Brown lists numerous compounds which are suggested for use as the further agent, including traditional chemotherapy agents such as cisplatin, hormones, various miscellaneous agents as well as immunosuppressive drugs. In the latter category, it is suggested that sulfasalazine may be used in the method of the invention. The further agent would be administered by the conventional route for the agent, which in the case of sulfasalazine would be oral administration. However, neither Brown nor the literature to date report any efficacy of sulfasalazine in the *in vivo* treatment of a tumor.

Andrianopoulos, G.D. *et al.* (1989) Anticancer Research 9:1725-1728, reported that daily oral ingestion of sulfasalazine by rats in doses equivalent to human daily doses did not significantly effect the incidence of colorectal tumors in the rats. Ritland *et al.* [supra] failed to show an anti-cancer effect through oral administration of sulfasalazine. Awasthi, *S. et al.* (1994) Br. J. Cancer 70:190-4, described *in vitro* administration of sulfasalazine to small-cell lung cancer cell lines treated with the chemotherapy agent, cisplatin. The results indicated that sulfasalazine enhanced the cytotoxicity of cisplatin by modulating cellular mechanisms involved in the cells acquiring cisplatin resistance. A further study involving administration of maximum tolerated oral doses of sulfasalazine to patients receiving the chemotherapy agent melphalan failed to demonstrate an increase in antitumor of the melphalan. (Gupta, *V. et al.* (1995) Cancer Chemother. Pharmacol. 36:13-19.)

Sulfasalazine is a N-heterocyclic substituted salicylate which includes a salicylic acid structure coupled at the 5' position by an azo bridge to a ring position in a benzenesulphonic acid which is amidated at the amine group of an amine-substituted heterocyclic ring which includes conjugated double bonds (see U.S. Patent No. 2,396,145). Sulfasalazine is not considered a NSAID but rather is a disease-modifying drug shown to be clinically effective in the treatment of autoimmune diseases such as rheumatoid arthritis and ulcerative colitis. Sulfasalazine inhibits activation and proliferation of T-lymphocytes and granulocytes. Sulfasalazine is administered orally and the azo bridge of the compound is cleaved through reductive action by microorganisms in the colon of a patient. The cleavage products are 5'-ASA (which is thought to be the effective agent in treatment of ulcerative colitis) and the antibiotic sulfapyridine (which is the cleavage product that is thought to be active in the treatment of rheumatoid arthritis).

It is desirable to obtain compounds that inhibit cellular uptake of cystine for the treatment of cyst(e)ine dependent tumors. Despite the evidence in the literature, it has now been found that a N-heterocyclic substituted salicylate such as sulfasalazine, is effective in inhibiting cellular uptake of cystine and is effective *in vivo* against cyst(e)ine-dependent tumors, in contrast to a simple salicylate such as sodium salicylate. It has also been discovered that in order to treat such cancers with N-heterocyclic substituted salicylates, the compound must be administered to the patient by means other than oral or rectal administration so as to avoid reductive cleavage, unless the compound is not subject to reductive cleavage in the gut of a patient.

### Summary of Invention

This invention provides the use of N-heterocyclic substituted salicylate compounds of Formula I (below) for the preparation of medicaments for use in the treatment of cancer, wherein the compounds are inhibitors of cellular uptake of cystine.

This invention also provides pharmaceutical compositions comprising a N-heterocyclic substituted salicylate compound that is an inhibitor of cellular uptake of cystine, incorporated into a liposome.

### Brief Description of the Drawings

Figure 1 is a graph showing the growth-inhibitory effects of NSAIDs used at therapeutic levels in rat lymphoma cultures.
Figure 2 is a graph showing the growth-inhibitory effects of sulfasalazine and its components, sulfapyridine and 5-aminosalicyclic acid (5-ASA) in rat lymphoma cultures.
Figure 3 is a graph showing growth of lymphoma transplants in rats as affected by intraperitoneal administration of sulfasalazine.
Figure 4 is a graph showing growth-inhibitory effects of sulfasalazine in cultures of rat lymphoma and human non-Hodgkin's cells.
Figure 5 is a graph showing the effect of a cystine or 2-ME on the growth-inhibitory activities of sodium salicylate and sulfasalazine in rat lymphoma cultures.

### Detailed Description

N-heterocyclic substituted salicylate compounds for use in the preparation of medicaments for the treatment of cancer are defined as comprising a salicylic acid or amide coupled to a ring position in a benzenesulphonic acid amidated by an amino group of an amine-substituted heterocyclic ring including conjugated double bonds. It should be understood that this definition. includes compounds in which the above-described components of the N-heterocyclic substituted salicylate are substituted with or conjugated to other moieties, providing the compound is capable of inhibiting cellular uptake of cystine.

Preferred N-heterocyclic substituted salicylate compounds are defined below by adapting the definition for some such compounds found in United States Patent No. 5,556,855. Such preferred compounds have the structure of Formula I:

Het-NR-SO₂-Ph¹-A-Ph²(-COR¹)(-OR²) (I)

including tautomeric forms, salts and solvates. Variants of each part of Formula I are described below and it is to be understood that each part may be varied in accordance with this description, independent of any other part. Thus, all possible combinations of such variants are contemplated for use

In Formula I, Het is a heterocyclic ring; Ph ¹ is a benzene ring; Ph² (COR¹)(OR²) is a benzene ring with the indicated substituents being preferably ortho to one another; R may be hydrogen or a lower alkyl; and, A is a bridging moiety. Het, Ph¹, Ph² (-COR¹) (-OR²) and A may be further substituted. R¹ may be -OH (such as in salicylic acid) or -NH₂ (such as in salicylamide), which substituents may in turn be substituted, preferably by a lower alkyl. R² may be hydrogen, or a lower acyl. Preferably, A is joined 5' to the -COR¹ substituent of Ph².

Bridging moiety A may be, or include a nitrogen-nitrogen bond (e.g. azo), a disulfide bridge, or an As=As bridge. Alternatively, A may be a bridge that is stable against hydrolysis and/or reduction in biological systems, in which case A is preferably a carbon chain, preferably a straight chain having from one to three carbon atoms which includes a carbon-carbon single, double or triple bond, optionally together with an oxo-substituent (=O) on one of the carbon atoms in the chain. A may be: -C≡C-; or, an unsubstituted or lower alkyl substituted, trans or cis-CH=CH-, -CH₂-CH₂-, -CO-CH=CH-, -CH=CH-CO-, -CO-, -CH₂-CO-, or -CH₂- (preferably -C≡C- or trans-CH=CH-). By stable against hydrolysis and/or reduction, it is meant that A lacks a reductive bond (such as azo) or a hydrolysis labile bond (such as an ester or amide bond), as a linking structure between Ph¹ and Ph².

Throughout this specification, the terms "lower alkyl", "lower alkoxy", and "lower acyl" mean groups which contain 1-6, 1-6, and 1-7 carbon atoms, respectively, optionally substituted with substituents such as any of R² - R⁵ described below.

In specific embodiments: Ph¹ is a 1,4- or 1,3- substituted benzene; Ph² (-COR¹) (-OR²) is an ortho-carboxy-hydroxy substituted phenyl, which may be further substituted with halogen or lower alkyl (preferably methyl) in its 3, 4 or 6 position; and/or, A is azo or a carbon chain such as: -C≡C-, -CH=CH-, -CH₂ CH₂-, -CO-CH=CH-, -CH= CHCO-, or -CH₂ CO-.

In specific embodiments, the heterocyclic ring in Het is five-membered or six-membered having two and three conjugated double bonds, respectively. Also, the heterocyclic ring in Het may be a monocyclic or bicyclic structure. Preferably, Het- is:

(R³, R⁴, R⁵)-Het'-

wherein Het' is: 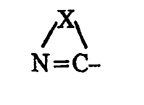 in which the free valency of Het' binds to NR in Formula I, and X is: (i) -N=CH-NH-, -N=CH-S-, -N=CH-O-, -NH-N=CH, -O-CH=CH-, -CH=CH-O-, -NH-CH=CH-, -CH=CH-NH-, -CH=CH-S-, -CH=N-NH-, -CH=CH-CH=CH-, -CH=CH-CH=N-, -CH=N-CH=CH-, -CH=CH-N=CH-, or -N=CH-CH=CH-, in which mutually adjacent hydrogen atoms (where possible), may be substituted in pairs with -CH=CH-CH=CH-, so as to form a bicyclic structure.

R³, R⁴, R⁵ are substituents on carbon atoms in Het'. The substituents may be independently hydrogen, lower alkyl, halogen, hydroxy, cyano, carboxy, lower alkoxy, benzyloxy, lower acyl (including acetyl, benzoyl, phenyl, benzyl, etc.), wherein any benzene rings that occur may also be substituted, preferably with lower alkyl. An example of a compound in which Het' is so substituted is the compound disalazine described in U.S. Patent 5,175,264. Disalazine is also an example of a compound in which Ph² (-COR¹)(-OR²) comprises an alkyl ester as contemplated above, since Ph² (-COR¹) (-OR²) is an acetysalicylic acid moiety. '

In specific embodiments, Ph² may be C₆ H₂ R⁶, where R⁶ is hydrogen, hydroxy, halogen or lower alkyl (preferably hydrogen, hydroxy or methyl).

Compounds described herein include tautomeric forms of compounds of Formula I. Compounds for use in this invention include pharmaceutically acceptable salts and solvates of the compounds described above. For example, compounds for use in this invention include salts with alkali metals (preferably sodium), salts with calcium or magnesium, complexes with pharmaceutically acceptable amines, and solvates with water, acetone, ethanol, etc. Use of a pharmaceutical composition comprising a compound (or a salt or solvate thereof) for use in this invention, is included in this invention.

Specific embodiments of compounds for use in this invention include sulfasalazine, disalazine, and salazosulfadimidine.

Compounds described herein may be joined to moieties which are intended to enhance the activity of the compound or increase tolerance by the patient of the compound. An example of such a moiety which may be covalently joined to a compound of Formula I, is a spacer susceptible to chemical hydrolysis joined to a glycosyl radical susceptible to cleavage by enzymatic hydrolysis, such as is described in United States Patent No. 6,020,315 (Bosslet *et al.*). Pharmaceutical compositions comprising a compound of Formula I joined to a spacer and glycosyl radical as described by Bosslet *et al*. may include a sugar or sugar alcohol ingredient.

Cancers that may be treated with the compositions disclosed herein include those in which neoplastic cells require extracellular cyst(e)ine. In this specification, and in contrast to any different meaning in the prior literature, a *cys*^{*-*} cancer is defined as a cancer in which neoplastic cells require extracellular cyst(e)ine. In other words, the neoplastic cells are cyst(e)ine dependent. These include lymphoid cancers (B and T cell type): including lymphoblastic lymphomas such as those presented in non-Hodgkin's disease, Burkitt's lymphoma (B and T cell type), lymphoblastic leukemias and lymphocytic leukemias; myeloid cancers, including: myelocytic leukemias; cyst(e)ine dependent solid tumors, including: melanomas, neuroblastomas; cancers involving lung fibroblasts; gliomal cancers; and, mammary cancers.

The literature describes various forms of cancer in which the neoplastic cells are cyst(e)ine dependent. Furthermore, neoplastic cells may be assessed for cyst(e)ine dependence by known means, for example by determining whether cultured cells fail to grow or exhibit reduced growth in the absence of cysteine or cystine, or will grow in the presence of a thiol such as 2-ME, or which express the X_{c}⁻ transport system (see: Sato, H. *et al.* (1997) J. Immunol. 158:3108-3117). Inhibition of growth or proliferation of neoplastic cells after administration of a compound for use in this invention, is also indicative of cyst(e)ine dependence of the neoplastic cells.

Some neoplastic cells have an intrinsic X_{c}⁻ transport system. In such cases, treatment according to this invention will directly inhibit cystine uptake by such cells. In the body, these cancer cells may still be able to pick up cysteine secreted by neighbouring cells. However, the anti-cancer effect of this invention *in vivo* is also brought about by inhibiting X_{c}⁻ system-mediated cystine uptake by neighbouring body cells (e.g. macrophages) thereby limiting availability of cysteine secreted by these cells in the environment of the cancer cells.

Compounds described herein may be administered by any known route of administration, but depending on the compound selected for use, the route of administration may have to be selected to prevent or limit exposure of the compound to the patient's gut. Rectal or oral administration is to be avoided unless the compound for use in this invention is such that it contains a bridge (e.g. bridging moiety A in Formula I) that will not be cleaved in the patient's gut. Preferred forms of administration are by intravenous or intraperitoneal injection for such cleavable compounds.

Compounds described herein may be formulated for oral, rectal, dermal, intraperitoneal and intravenous administration by any means known in the art, including preparation of solutions of compounds of Formula I (or the salts thereof) in a suitable solvent such as water, saline, ethanol, or acetone; or, by preparation of solutions for injection in which the compounds of Formula I have been solubilized through the use of a pharmaceutically acceptable co-solvent such as DMSO. When a compound for use in this invention is suitable for oral administration, the compound may be formulated for oral or rectal administration by any means known in the art, including admixing the compound with one or more pharmaceutically suitable carriers, such as those known for administration of sulfasalazine, the N-heterocyclic substituted salicylic acids described in United States Patent No. 5,556,855, and the preparations and dosage forms described in United States Patent No. 6,020, 315,

Compounds described herein may be administered in conjunction with other anti-cancer therapies including administration of radiation or administration of anti-neoplastic drugs. Such combined treatment may increase the effectiveness of the individual therapies. For example, compounds for use in this invention may be administered in conjunction with any of a variety of therapeutic or chemotherapy agents, including cytotoxic drugs such as those which target proliferating cells, hormones or hormone antagonists, monoclonal antibodies (e.g. rituximab) and nucleic acid based therapeutics such as anti-sense nucleic acids intended to alter expression of cancer related genes. Pharmaceutical compositions may be formulated to deliver both a compound for use in this invention as well as another anti-neoplastic drug or agent. For example, liposomes (e.g. anionic liposomes, see WO 99 / 30686) could be used to deliver both a nucleic acid based therapeutic and a compound for use in this invention. Liposomes having surface groups such as antibodies may be formulated to target neoplastic cells or macrophages to deliver a compound for use in this invention which is incorporated into the liposome. Liposomes may be formulated to incorporate both a compound for use in this invention as described above and a chemotherapy drug such as an alkylating agent.

It may be particularly advantageous to combine the compositions described herein with anti-neoplastic drugs which are intended to lead to oxidative stress in neoplastic cells. Examples of such drugs are alkylating agents such as cyclophosphamide and chlorambucil. This is because apoptosis of cysteine-starved cells may result in deficiency in cellular glutathione (a free radical scavenger), which may in turn make the cell more susceptible to oxidative stress.

Normal lymphoid cells could be protected, during treatment of γ-cystathionase-deficient cancers in accordance with this invention, by concurrent administration of L-cystathionine. This would permit the normal cells to continue to make cysteine.

For *in vivo* treatment of a mammal (animal or human), the frequency and amount of administration of a compound for use in this invention will be determined by the practitioner in order to achieve blood circulation levels of the intact compound which are effective for the treatment. Preferably, the plasma level of a compound for use in this invention to be achieved or maintained will be in the range of 0.05 - 0.5mM unless formulations are used to enhance delivery or circulation lifetime of the compound.

Previously, N-heterocyclic substituted salicylates were intended for oral administration. The discovery that such compounds have utility when delivered directly (e.g. into the bloodstream) to neoplastic cells and macrophages provides the basis for novel pharmaceutical formulations of the compounds described herein. These formulations facilitate the delivery of the compounds via the bloodstream. Liposome formulations are ideally suited for this purpose. Compounds for use in this invention may be readily incorporated into liposomes using current methodologies. Furthermore, liposome formulations are available that have increased blood circulation time which facilitates delivery of incorporated agents to target cells and permits a reduction in the amount and frequency of administration.

The term "liposome" as used herein means a vesicle comprised of one or more concentrically ordered lipid bilayers encapsulating an aqueous phase. Formation of such vesicles requires the presence of vesicle forming lipids, which are amphipathic lipids capable of assuming or being incorporated into a bilayer structure. This includes lipids that are capable of forming a bilayer by itself or when in combination with another lipid or lipids. An amphipathic lipid is incorporated into a lipid bilayer by having its hydrophobic moiety in contact with the interior, hydrophobic region of the bilayer membrane and its polar head moiety oriented towards an outer, polar surface of the membrane. Hydrophilicity arises from the presence of functional groups such as hydroxyl, phosphato, carboxyl, sulfato, amino or sulfhydryl groups. Hydrophobicity results from the presence of a long chain of aliphatic hydrocarbon groups.

Vesicle-forming lipids that may be incorporated into liposomes or lipid carriers of this invention may be selected from a variety of amphiphatic lipids, typically including phospholipids such as phosphatidylcholine, phosphatidylethanolamine, phosphatidic acid, phosphatidylinositol, or phosphatidylglycerol; sterols such as cholesterol; and, sphingolipids such as sphingomyelin.

Liposomes designed for increased circulation time in the bloodstream may comprise a hydrophilic polymer-lipid conjugate. This term refers to a vesicle-forming lipid covalently joined at its polar head moiety to a hydrophilic polymer, and is typically made from a lipid that has a reactive functional group at the polar head moiety in order to covalently attach to the hydrophilic polymer. Suitable reactive functional groups are for example an amino, hydroxyl, carboxyl, or formyl group. The lipid in a hydrophilic polymer-lipid conjugate may be any lipid described in the art for use in such conjugates and is preferably a phospholipid such as phosphatidylcholine, phosphatidylethanolamine, phosphatidic acid or phosphatidylinositol, having two acyl chains with varying length and degrees of unsaturation. The hydrophilic polymer is typically a biocompatible polyalkyether characterized by a solubility in water that permits the polymer chains to effectively extend away from the liposome surface out into the aqueous shell surrounding the liposome and a flexibility of the chains that produces a uniform surface coverage of the liposome. Suitable hydrophilic polymers include polyethylene glycol (PEG), polymethylethylene glycol, polyhydroxypropylene glycol, polypropylene glycol, polylactic acid, polyglycolic acid, polylactic/polyglycolic acid copolymers, or polyacrylic acid as well as those disclosed in United States Patent 5,013,556 and 5,395,619. Preferably, the hydrophilic polymer is PEG having a molecular weight between about 500 and 5000 Daltons.

Liposome compositions of the present invention may be generated by a variety of known techniques including lipid film/hydration, reverse phase evaporation, detergent dialysis, freeze/thaw, homogenization, solvent dilution and extrusion procedures. Any hydrophilic surface polymers may be incorporated as one of the lipid components at the time of initial liposome formation or may be added subsequent to liposome formation by incorporation through lipid exchange or derivatization of the liposome surface. Bioactive agents may be encapsulated inside the liposomes of this invention by passive or active loading methodologies known in the art.

Liposomes of the present invention for parenteral or intravenous administration are preferably formulated in a sterile aqueous solution which may comprise excipients known to be tolerated by warm-blooded animals. For oral or topical applications, liposome compositions of the present invention may be incorporated in vehicles commonly used for such applications such as creams, salves, ointments and slow release patches for topical medical applications and tablets, capsules, powders, suspensions, solutions and elixirs for oral applications.

When this invention is employed to inhibit cellular uptake of cystine or to assess neoplastic cells *in vitro,* compounds of this invention may be added to a growth medium suitable for maintaining the cells or tissues being treated.

### Example 1: Comparison of NSAIDS and Salicylates

### Drugs

Indomethacin, piroxicam, ibuprofen, aspirin and salicylamide were solubilized in dimethylsulfoxide (DMSO) and tested for culture growth-inhibitory properties as solutions in culture medium (pH ca. 7.5) containing 0.2% DMSO, a vehicle concentration which did not interfere with culture growth. Solubilization of salicylhydroxamic acid and sulfasalazine involved use of 0.1 N NaOH and adjustment of the pH to 7.5-7.7 using 1 N HCl, which was added slowly to the solution swirled by a magnetic stirrer. Sodium salicylate dissolved readily in culture medium. All drugs were obtained from Sigma-Aldrich Canada Ltd., Oakville, ON, Canada. Drug solutions and vehicles (controls) were prepared and assayed under subdued light conditions.

### Cell Cultures

Nb2 lymphoma is of a pre-T cell origin. The parental line (Nb2 U17) is non-metastatic as subcutaneous implants whereas subline Nb2-SFJCD1 is highly metastatic. Nb2-SFJCD1 cultures (doubling time = 12 - 13 hr) were maintained in Fischer's medium (Sigma-Aldrich), supplemented with 10.0 % horse gelding serum (lactogen-deficient; one batch, ICN Biomedicals, Inc., Auroro, OH), penicillin (50 U/ml) and streptomycin (50 µg/ml). In this medium the cells grow as clumps which can be readily dispersed. Nb2 lymphoma cell lines are used by a number of laboratories; the Nb2-11 subline, is available from the European Collection of Animal Cell Cultures (ECACC) in Salisbury, U.K. The Nb2-U17 and Nb2-SJFCD1 cell lines are available from Dr. Peter W. Gout, B.C. Cancer Research Centre, Vancouver, B.C., Canada. DoHH2 cell suspension cultures (doubling time ca. 20 hours) were maintained in RPMI-1640 medium (Stem Cell Technology, Vancouver, B.C., Canada) containing fetal bovine serum (5%), horse serum (5%) and antibiotics.

### Culture Growth Inhibition Assays

Nb2-SFTCD1 cells from log phase cultures were centrifaged (3.5 min at 350xg) and gently resuspended in fresh Fisher's medium containing 10% horse gelding serum. Aliquots (1.80 ml) were distributed in 12-well tissue culture plates (Linbro, Flow Laboratories, Mississauga, ON, Canada) and preincubated for 3 - 5 hours at 37°C in a water-saturated 5% CO₂/air atmosphere. Solutions of the drugs (200 µl portions) were then added to the cultures for a 45 hour incubation (Cin = 1.0x10⁵ cells/ml). Cell populations were determined using an electronic counter (Coulter Electronics, Hialeah, FL). Culture growth inhibitions were calculated from the differences in population increase found between drug-treated cultures and their controls.

Assays using DoHH2 cells were similarly performed, using their maintenance medium; Cin = ca. 1.5x10⁵ cells/ml (see: Kluin-Nelemans, H.C. *et al.* (1991) Leukemia 5:221.)

### Assay of Tumor Growth Inhibition in Rats

A colony of Nb rats is maintained in the British Columbia Cancer Research Centre Vancouver, B.C., Canada. Approximately equal portions of minced Nb2-U17 tumor tissue, developed subcutaneously in a rat following injection of cultured Nb2-U17 cells, were injected by trocar subcutaneously in the nape of the neck of groups of male Nb rats (one injection per rat), that had been lightly anesthetized with isoflurane (Abbott Laboratories Ltd., Montreal, Canada). When the single tumors reached a measurable size (range used: 0.3-1.8 g), drug therapy of 350-400 gr animals was started, using oral administration (by stomach tube; sulfasalazine) or intraperitoneal (i.p.) injections (sodium salicylate, sulfasalazine) at a site remote from the transplant, at approximately 12 hour intervals. Fresh drug solutions were prepared every day in 2% carboxy-methylcellulose (CMC) for oral administration or, for i.p. injections, in saline (sodium salicylate) or in 0.1 N NaOH (sulfasalazine) subsequently adjusted to pH 8.0 using 1 N HCl. 2 % CMC or saline were used for controls. Drug preparation and administration were carried out under subdued light conditions. Food and water were provided ad libitum. Tumor size, body weight and general health of the rats were monitored twice a day. Tumor size was measured using calipers and expressed in grams using the formula: π/6 x length x width x height in cm. Animals were sacrificed by carbondioxide asphyxiation as required by the protocol or as soon as they showed signs of discomfort. On necropsy the animals were examined for evidence of metastatic spread with particular attention to tissues such as kidneys, liver and spleen, the target tissues of metastatic Nb2 lymphoma cells. Tissue sections were taken for histologic analysis. Non-tumor-bearing animals were used to establish maximally tolerated dosages.

### Results

A variety of NSAIDs, spanning a wide range of anti-inflammatory potency, were tested for growth-inhibitory activity in the Nb2 cell cultures as well as various salicylates. The NSAIDs included indomethacin, a potent inhibitor of prostaglandin forming cyclooxygenase, and sodium salicylate, considered to be one of the weakest NSAIDs. The drugs were used at a range of concentrations spanning their "therapeutic levels," as reported for plasma of patients undergoing treatment for inflammatory diseases. With increasing concentrations, all the NSAIDs tested were able to abrogate population growth. The drugs were also tested using cultures containing 2-mercaptoethanol at 50-90 µM. At this concentration range the thiol circumvents specific inhibition of the x_{c}⁻ system as can be obtained with monosodium glutamate. However, the presence of 2 mercaptoethanol in the cultures did not lead to a decline in NSAID-induced growth inhibition showing that the growth-inhibitory action of the NSAIDs in the lymphoma cell cultures was not primarily a result of inhibition of the x_{c}⁻ system.

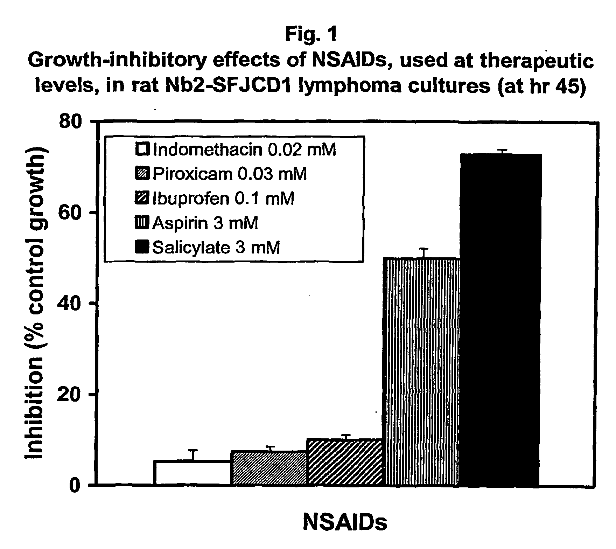

Table 1A shows the IC50s obtained for selected NSAIDs, presented in order of decreasing anti-cyclooxygenase activity versus their reported therapeutic levels in plasma of patients. The IC50s of indomethacin, piroxicam and ibuprofen markedly exceeded their therapeutic levels, in contrast to the IC-50s of the salicylate NSAIDs, i.e. aspirin (acetylsalicylic acid) and sodium salicylate, which were in the same range as their therapeutic levels. Fig. 1 illustrates the differential in the growth-inhibitory potencies of the drugs when used at "therapeutic concentrations." Indomethacin (0.02 mM), piroxicam (0.03 mM) and ibuprofen (0.1 mM) inhibited the growth of the Nb2 cell cultures to only a minor extent, in the range 5-10%. In contrast, aspirin (3 mM) and, in particular, sodium salicylate (3 mM), inhibited the growth of the cultures to a greater extent, i.e. by ca. 50 and 70 % , respectively. With regard to the action of aspirin it is notable that salicylic acid is a major metabolite of aspirin, generated by its hydrolysis or following the acetylation by aspirin of substrates such as COX-1. Thus, the growth-inhibitory action of aspirin in the lymphoma cell cultures may be due to its conversion to salicylate.

Table 1B presents results for various non-NSAID salicylate derivatives, including modification of the carboxyl group of salicylic acid. In particular, salicylhydroxamic acid and sulfasalazine, possessed highly elevated growth-inhibitory potencies relative to sodium salicylate. As shown in Table 1B, sulfasalazine was found to be a highly potent inhibitor of Nb2 lymphoma culture growth, showing an IC50 of ca. 0.17 mM, a concentration within the range of its therapeutic levels (0.08 - 0.2 mM). Furthermore, whereas sulfasalazine at 0.15 mM only partially arrested growth during the course of a 45 hour experiment, a slightly higher concentration, i.e. 0.2 mM, induced severe cell lysis in the period 24-45 hours.

**Table 1**

| Growth-inhibitory activities of NSAIDs and salicylate derivatives in Nb2-SFJCD1 cultures and reported therapeutic levels in plasma | | |
|---|---|---|
| Drug | IC50 (mM)^{a} Average ± S.D. | Therapeutic drug levels in plasma (mM)^{b} |
| A | | |
| Indomethacin | 0.15 ± 0.01 | 0.005 - 0.02 |
| Piroxicam | 0.30 ± 0.02 | 0.015 - 0.03 |
| Ibuprofen | 0.43 ± 0.01 | 0.05 - 0.1 |
| Acetylsalicylic acid | 3.09 ± 0.28 | 1-3 |
| Salicylate, sodium | 1.82 ± 0.12 | 1-3 |

| B | | |
|---|---|---|
| Salicylamide | 1.34 ±0.13 | n.a. |
| Salicylhydroxamic acid | 0.13 ± 0.02 | n.a. |
| Sulfasalazine | ca. 0.17 | 0.08 - 0.2 |

| | | |
|---|---|---|
| ^{a}Nb2-SFJCD1 rat lymphoma cells, resuspended in fresh Fischer's medium containing 10.0% horse gelding serum, were incubated with a drug for 45 h at 37°C in a 5% CO2 atmosphere; growth inhibitions were calculated from cell number increases relative to those in control cultures (no drug). | | |
| ^{b}As reported in the literature for patients/controls undergoing treatment with the drugs. | | |

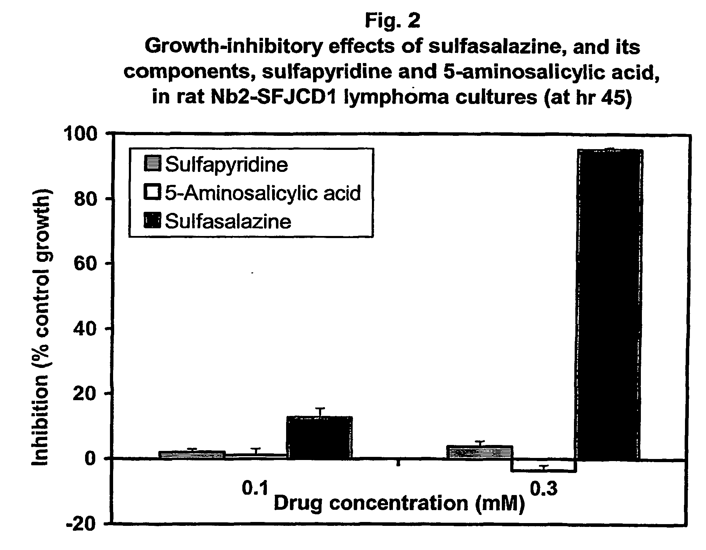

A comparison of the growth-inhibitory activities of sulfasalazine and its components (Fig. 2) showed that whereas sulfasalazine was highly potent in the range 0.1 - 0.3 mM, neither of its cleavage products sulfapyridine, nor 5-aminosalicylic acid, had any growth-inhibitory activity at this concentration range, which covers the therapeutic plasma levels of both sulfapyridine (0.08 - 0.2 mM) and 5-aminosalicylic acid (ca. 0.01 mM). Thus, the lymphoma growth-inhibitory activity of sulfasalazine is based on its action as an intact molecule.

Sodium salicylate and sulfasalazine were evaluated for inhibition of experimental rat lymphoma growth in Nb rats. Groups of male rats (350-400 gr) were used, each carrying a single, well-developed, rapidly growing, non-metastatic, subcutaneous Nb2-U17 tumor transplant in the nape of the neck. Non-metastatic Nb2-U17 tumors were employed to avoid dissemination of cells from the tumor which would interfere with measurements of drug-induced changes in tumor mass. It was established *in vitro* that the Nb2-U17 cell line was as sensitive to salicylate-induced growth inhibition as the Nb2-SFJCD1 cell line. The animals were treated for a 7-day period, with drug administration taking place at approximately 12 hour intervals in an attempt to maintain elevated levels of the drug in the circulation. It was found that administration of sodium salicylate to the animals (n = 4) did not inhibit tumor growth, even though a relatively high dosage was used (i.p. 150 mg/kg body wt, b.i.d.). Oral administration of sulfasalazine (500 mg/kg body wt, b.i.d.), had only minor lymphoma growth-inhibitory effect.

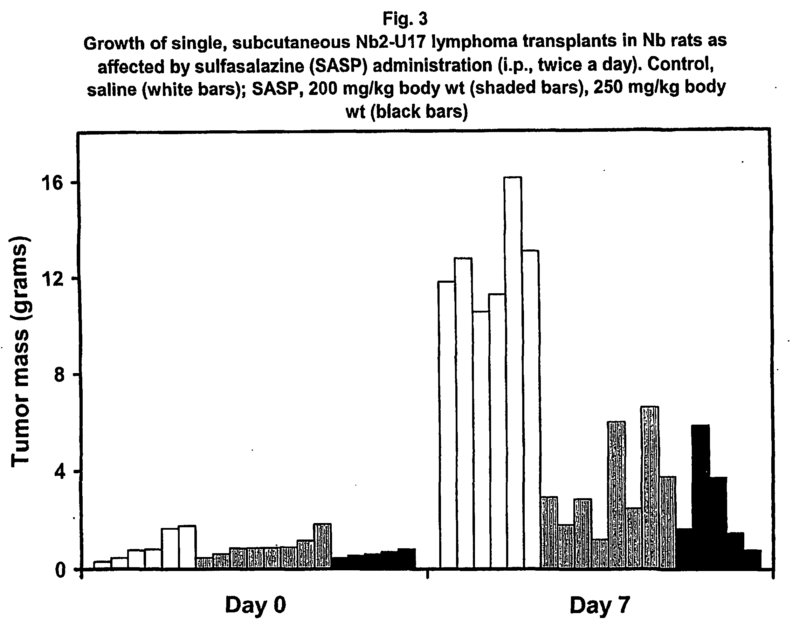

Sulfasalazine was administered to rats intraperitoneally, to avoid its cleavage in the gut. As shown in Fig. 3, the tumor transplants in saline-treated control rats (n = 6) grew rapidly from an average of 0.95 gr to an average of 12.6 gr in 7 days, consistent with the usual rapid growth of Nb2-U17 transplants. In contrast, the growth of the tumors in the two groups of rats (total n = 13), treated with sulfasalazine at very high but well-tolerated dosages, was in all cases substantially inhibited. The tumors in the rats treated with sulfasalazine at the lower dose (200 mg/kg body wt, b.i.d.; n = 8) showed an increase from an average size of 0.93 gr to an average size of only 3.43 gr. This amounts to an average growth inhibition, relative to the controls, of approximately 80%. Similarly, the tumors in the rats treated with sulfasalazine at a maximally tolerated dose (250 mg/kg body wt, b.i.d.; n = 5) increased from an average size of 0.60 gr to an average size of only 2.65 gr. In three of the 13 cases, sulfasalazine-induced growth suppression amounted to 90-100%.

Necropsy of sulfasalazine-treated, tumor-bearing animals showed that the drug had not induced metastasis. Reduced tumor growth in the animals was therefore not the result of drug-induced dissemination of tumor cells from the transplant site, but reflected genuine, tumor growth inhibition. The drug had no major side effects, even at the higher dose, as indicated by the general health and appearance of the tumor-bearing rats (n = 13) during treatment and at necropsy. During therapy the animals remained active and their eyes stayed bright; there was no evidence of diarrhea, or changes in the appearance of their fur. A minor side effect was found in the thickening of tissue at the site of the injections. Control, non-tumor-bearing animals (n = 2) recovering an identical 7-day treatment with the drug and followed over a three months period, showed only a temporary halt in their weight gain; the scar tissue at the site of injections disappeared within a few weeks.

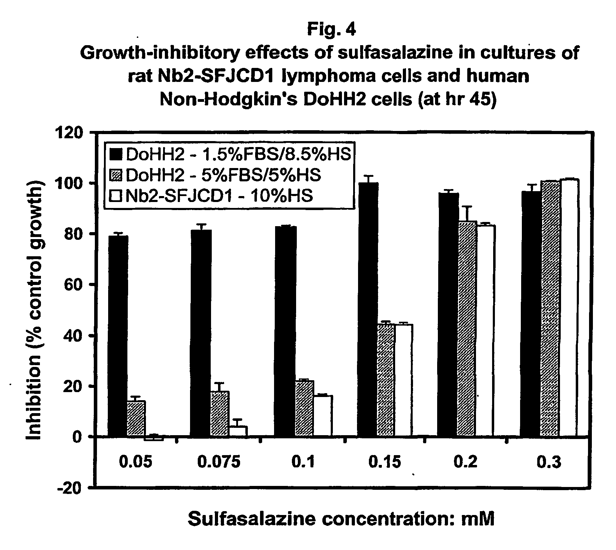

Sulfasalazine also demonstrates marked *in vitro* growth-inhibitory activity with regard to DoHH2 cells, a line of cultured human B-cell non-Hodgkin's lymphoma cells. In their regular culture medium, containing 5% fetal bovine serum (FBS) + 5% horse serum (HS), these cells displayed a sensitivity to the drug which was similar to that of rat Nb2 lymphoma cells (cultured in medium containing 10% HS) (Fig. 4). The growth of DoHH2 cell cultures is dependent on the presence of FBS in the culture medium, presumably due to growth factors present in this serum. When the amount of FBS in the culture medium of the DoHH2 cells was reduced to 1.5% FBS, the doubling time of the control cultures increased from about 20 to 29 hr. In addition, the cells showed a much higher sensitivity to sulfasalazine, displaying extensive cell lysis even at the low drug concentration of 0.05 mM (at hr 45) (Fig. 4).

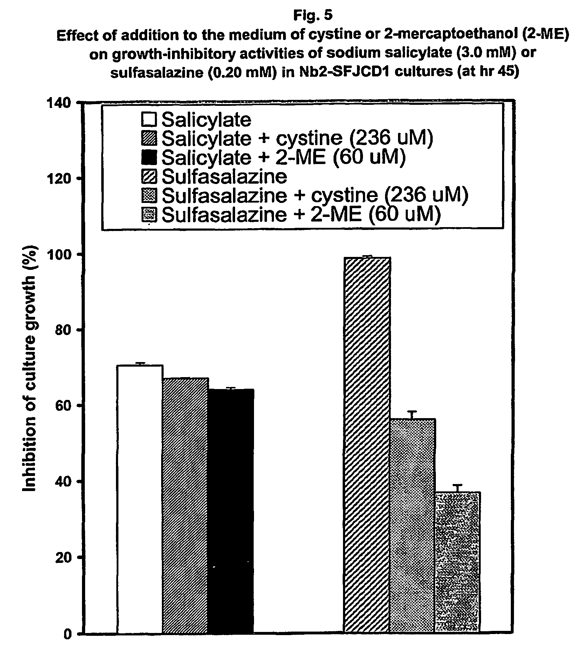

The anti-lymphoma effect of sulfasalazine is related to inhibition of cellular uptake of the amino acid, cystine. This process is mediated in Nb2-SFJCD1 cells by the X_{c}⁻. system. As shown in Fig. 5, the growth-inhibitory effect of sulfasalazine was substantially reduced by the presence in the culture medium of cystine at ca. 3-fold elevated levels and even more so by 2-mercaptoethanol (by ca. 63 % at 60 µM). In contrast, the growth-inhibitory effect of sodium salicylate was only marginally affected by the increased cystine levels (Fig. 5).

In summary, only sulfasalazine and sodium salicylate exhibited *in vitro* potency within the range of their reported therapeutic levels in patients. The cleavage products of sulfasalazine (sulfapyridine and 5-ASA) were devoid of growth-inhibitory activity *in vitro.* However, intraperitoneal administration of sulfasalazine to Noble (Nb) rats markedly inhibited the growth of single, well-developed, rapidly growing, non-metastatic, subcutaneous Nb2 lymphoma transplants during a seven day trial, with an average inhibition of 80 % relative to controls. In roughly 1/4 of the cases, inhibition amounted to 90-100%. Intraperitoneal administration of sodium salicylate had no effect. Further, sulfasalazine markedly inhibited the growth of cultures of DoHH2 cells (human B cell, non-Hodgkin's lymphoma). Thus, sulfasalazine is a potent inhibitor of lymphoma cell proliferation and results in tumor cell lysis. The anti-tumor effect of sulfasalazine, in contrast to sodium salicylate, relates to inhibition of cellular uptake of cystine.

### Example 2: Assessment and Treatment of Cys Cancer Cells

A study was conducted to determine whether estrogen-receptor positive MCF-7 or the highly progressed, receptor negative MDA-MB-231 human breast carcinoma cell lines express the xc⁻ transporter and whether such cells are susceptible to treatment with sulfasalazine. Using an RT-PCR assay to screen for expression of a xc⁻ protein, each of the cell lines was found to express the transporter, with higher levels observed in the MDA-MB-231 line. The effect of sulfasalazine on cell proliferation was subsequently assessed. Sulfasalazine, but not its colonic metabolites, inhibited growth of each cell line in a concentration-dependent manner with an IC₅₀ of 0.5±0.1 and 0.3 ±0.005 mM for MCF-7 and MDA-MB-231 cells, respectively. Addition of 2-ME (60µM) completely antagonized the inhibitory actions of sulfasalazine in MDA-MB-231 cells, while the thiol was only 50% effective as an antagonist in the MCF-7 line. Monosodium glutamate (10 mM) which selectively antagonizes xc⁻, inhibited proliferation in MCF-7 cells to an extent similar to that observed for sulfasalazine; addition of 2-ME to the cultures completely negated this effect. Thus, the xc⁻ transporter is a target for the anti-cancer actions of sulfasalazine in human breast carcinoma.

## Claims

1. The use of a N-heterocyclic substituted salicylate compound or pharmaceutically acceptable salt or solvate thereof for preparation of a medicament for the treatment of cancer, the compound having the formula:
Het-NR-SO₂-Ph¹-A-Ph²(-COR¹)(-OR²)
wherein:
Het is an optionally substituted heterocyclic ring, wherein the ring is five-membered or six-membered having two and three conjugated double bonds, respectively or a mono cyclic or a bicyclic structure, wherein Het- is (R³, R⁴, R⁵)-Het'- and wherein Her is: 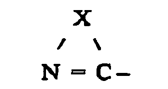 in which the free valency of Her binds to NR in Formula I, and X is selected from: -N=CH-NH-, -N=CH-S-, -N=CH-O-, -NH-N=CH, -O-CH=CH-, -CH=CH-O-, -NH-CH=CH-, -CH=CH-NH-, -CH=CH-S-, -CH=N-NH-, -CH=CH-CH=CH-, -CH=CH-CH=N-, -CH=N-CH=CH-, -CH=CH-N=CH-, or -N=CH-CH=CH-, in which mutually adjacent hydrogen atoms are optionally substituted in pairs with -CH=CH-CH=CH-, so as to form a bicyclic structure, and R³, R⁴ and R⁵ are substituents on carbon atoms in Het' as defined below;
Ph¹ is an optionally substituted benzene ring;
Ph²(COR¹)(OR²) is an optionally substituted benzene ring, wherein Ph² is an ortho-carboxy-hydroxy substituted phenyl optionally substituted with halogen or 1-6 carbon alkyl further optionally substituted with substituents such as any of R² - R⁵ in its 3, 4 or 6 position;
A is selected from the group consisting of: -N=N-; -S-S-; -As=As-; a straight chain having from 1-3 carbon atoms and having either a carbon-carbon single, double or triple bond optionally together with an oxo-substituent (=O) on one of the carbon atoms in the chain; -C≡C-; an unsubstituted or 1-6 carbon alkyl substituted, trans or cis -CH=CH-, -CH₂-CH₂-, -CO-CH=CH-, -CH=CH-CO-, -CO-, -CH₂-CO-, or-CH₂-;
R is selected from hydrogen or a 1-6 carbon alkyl optionally substituted with substituents selected from R² - R⁵;
R¹ is -OH or -NH₂, and optionally substituted by a 1-6 carbon alkyl optionally substituted with any of R² - R⁵;
R² is hydrogen, or a 1-7 carbon acyl optionally substituted with any of R²- R⁵; and
R³, R⁴, R⁵ are independently hydrogen, 1-6 carbon alkyl, halogen, hydroxy, cyano, carboxy, 1-6 carbon alkoxy, benzyloxy, 1-7 carbon acyl, wherein any benzene rings are optionally substituted with 1-6 carbon alkyl.

2. The use of claim 1, wherein Ph¹ is benzene.

3. The use of claim 1, wherein Ph² is C₆H₂R⁶ and R6 is hydrogen, hydroxy, halogen or a 1-6 carbon alkyl.

4. The use of claim 1, wherein A is selected from -N=N-; -C≡C-; -CH=CH-; -CH₂-CH₂ ; -CO-CH=CH-; -CH=CHCO-, or -CH₂-CO-.

5. The use of any one of claims 1-4, wherein the cancer is *cys*^{*-*}*.*

6. The use of any one of claims 1-5, wherein the salicylic acid or amide is coupled to the ring position by a bridging moiety which is stable against hydrolysis or reduction in biological systems.

7. The use of any one of claims 1-6, wherein the compound is sulfasalazine, disalazine, or salazosulfadimidine.

8. The use of claim 7, wherein the compound is sulfasalazine.

9. The use of claim 7 or 8, wherein the compound is for intraperitoneal or intravenous administration.

10. The use of any one of claims 1-9, wherein the compound is incorporated into a liposome.

11. A pharmaceutical composition comprising the compound of claim 1 incorporated into a liposome.

12. The composition of claim 11, wherein the compound is a compound of claims 2, 3 or 4.

13. The composition of claim 12, wherein the compound is sulfesalazine, disalazine, or salazosulfadimidine.

14. The composition of claim 13, wherein the compound is sulfasalazine.

## Patentansprüche

1. Verwendung einer N-heterocyclischen substituierten Salicylatverbindung oder eines pharmazeutisch akzeptablen Salzes oder Solvats derselben zur Herstellung eines Medikaments für die Behandlung von Krebs, wobei die Verbindung die Formel aufweist:
Het-NR-SO₂-Ph¹-A-Ph²(-COR¹) (-OR²)
wobei:
Het ein optional substituierter heterocyclischer Ring ist, wobei der Ring 5-gliedrig oder 6-gliedrig mit zwei bzw. drei konjugierten Doppelbindungen oder eine monocyclische oder eine bicyclische Struktur ist, wobei Het- (R³, R⁴, R⁵) - Het'- ist und wobei Het' ist: 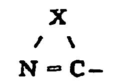 bei dem die freie Valenz von Het' an NR in Formel I bindet und X ausgewählt ist aus: -N=CH-NH-, -N=CH-S-, -N=CH-O-, -NH-N=CH, -O-CH=CH-, -CH=CH-O-, -NH-CH=CH-, -CH=CH-NH-, -CH=CH-S-, -CH=N-NH-, -CH=CH-CH=CH-, -CH=CH-CH=N-, -CH=N-CH=CH-, -CH=CH-N=CH- oder -N=CH-CH=CH-, bei denen zueinander angrenzende Wasserstoffatome optional paarweise mit -CH=CH-CH=CH- substituiert sind, um so eine bicyclische Struktur zu bilden und R³, R⁹ und R⁵ Substituenten an Kohlenstoffatomen in Het', wie unten definiert, sind;
Ph¹ ein optional substituierter Benzolring ist;
Ph²(COR¹)(OR²) ein optional substituierter Benzolring ist, wobei Ph² ein ortho-Carboxy-Hydroxy-substituiertes Phenyl ist, das optional mit Wasserstoff oder 1-6 Kohlenstoff-Alkyl substituiert ist, das optional weiter mit Substituenten wie etwa R²-R⁵ an seiner 3, 4 oder 6-Position substituiert ist;
A ausgewählt ist aus der Gruppe, die besteht aus: -N=N-, -S-S-; -As=As-; einer geraden Kette mit 1-3 Kohlenstoffatomen und mit entweder einer Kohlenstoff-Kohlenstoff-Einzel-, -Doppel- oder -Dreifachbindung, optional zusammen mit einem Oxo-Substituenten (=O) an einem der Kohlenstoffatome in der Kette; -C≡C-; einem unsubstituiertem oder 1-6 Kohlenstoff-Alkyl-substituiertem, trans oder cis -CH=CH-, -CH₂-CH₂-, -CO-CH=CH-, -CH=CH-CO-, -CO-, -CH₂-CO oder -CH₂-;
R ausgewählt ist aus Wasserstoff oder 1 bis 6-Kohlenstoff-Alkyl, optional substituiert mit Substituenten; die aus R²-R⁵ ausgewählt sind;
R¹ -OH oder -NH₂ ist und optional durch 1 bis 6 Kohlenstoff-Alkyl substituiert ist, das optional mit irgendeinem R²-R⁵ substituiert ist;
R² Wasserstoff oder 1 bis 7 Kohlenstoff-Acyl ist, das optional mit irgendeinem R²-R⁵ substituiert ist; und
R³, R⁴, R⁵ unabhängig voneinander Wasserstoff, 1-6 Kohlenstoff-Alkyl, Halogen, Hydroxy, Cyano, Carboxy, 1-6 Kohlenstoff-Alkoxy, Benzyloxy, 1 bis 7 Kohlenstoff-Acyl sind, wobei einer der Benzolringe optional mit 1 bis 6 Kohlenstoffa-Alkyl substituiert ist/sind.

2. Verwendung nach Anspruch 1, wobei Ph¹ Benzol ist.

3. Verwendung nach Anspruch 1, wobei Ph² C₆H₂R⁶ ist und R⁶ Wasserstoff, Hydroxy, Halogen oder ein 1-6 Kohlenstoff-Alkyl ist.

4. Verwendung nach Anspruch 1, wobei A ausgewählt ist aus -N=N-; -C≡C-; -CH=CH-; -CH₂-CH₂-; -CO-CH=CH-; -CH=CHCO- oder -CH₂-CO-.

5. Verwendung nach einem der Ansprüche 1 bis 4, wobei der Krebs cys⁻ ist.

6. Verwendung nach einem der Ansprüche 1 bis 5, wobei die Salicylsäure oder das Amid mit der Ringposition durch eine Brückeneinheit gekoppelt ist, die gegenüber einer Hydrolyse oder Reduktion in biologischen Systemen stabil ist.

7. Verwendung nach einem der Ansprüche 1 bis 6, wobei die Verbindung Sulfasalazin, Disalazin oder Salazosulfadimidin ist.

8. Verwendung nach Anspruch 7, wobei die Komponente Sulfasalazin ist.

9. Verwendung nach Anspruch 7 oder 8, wobei die Verbindung zur intraperitonealen oder intravenösen Gabe bestimmt ist.

10. Verwendung nach einem der Ansprüche 1 bis 9, wobei die Verbindung in ein Liposom inkorporiert ist.

11. Pharmazeutische Zusammensetzung, umfassend die in ein Liposom inkorporierte Verbindung von Anspruch 1.

12. Zusammensetzung nach Anspruch 11, wobei die Verbindung eine Verbindung der Ansprüche 2, 3 oder 4 ist.

13. Zusammensetzung nach Anspruch 12, wobei die Verbindung Sulfasalazin, Disalazin oder Salazosulfadimidin ist.

14. Zusammensetzung nach Anspruch 13, wobei die Verbindung Sulfasalazin ist.

## Revendications

1. Utilisation d'un composé salicylate à substitution N-hétérocyclique ou un sel ou solvate pharmaceutiquement acceptable de celui-ci pour la préparation d'un médicament pour le traitement du cancer, le composé ayant la formule :
Het-NR-SO₂-Ph¹-A-Ph²(-COR¹)(-OR²)
dans laquelle :
Het est un noyau hétérocyclique éventuellement substitué, dans lequel le noyau a cinq ou six chaînons ayant deux et trois doubles liaisons conjuguées, respectivement ou une structure monocyclique ou bicyclique, dans laquelle Het est (R³, R⁴, R⁵)-Het¹- et dans laquelle Het' est : 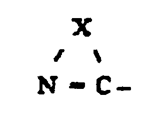 dans laquelle la valence libre de Het' se lie à NR dans la formule I, et X est choisi parmi : -N = CH-NH-, -N = CH-S-, -N = CH-O-, -NH-N = CH, -O-CH = CH-, -CH = CH-O-, -NH-CH = CH-, -CH = CH-NH-, -CH = CH-S-, -CH = N-NH-, -CH = CH-CH = CH-, -CH = CH-CH = N-, -CH = N-CH = CH-, -CH = CH-N = CH-, ou -N = CH-CH = CH-, dans lesquels des atomes d'hydrogène mutuellement adjacents sont facultativement substitués en paires avec -CH = CH-CH = CH-, de manière à former une structure bicyclique, et R³, R⁴, R⁵ sont des substituants sur des atomes de carbone dans Het' comme définis ci-dessous ;
Ph¹ est un noyau benzénique facultativement substitué ;
Ph²(COR¹)OR²) est un noyau benzénique facultativement substitué, dans lequel Ph² est un phényle à substitution ortho-carboxy-hydroxy facultativement substitué avec un halogène ou un alkyle de 1 à 6 carbones facultativement substitué en outre avec des substituants tels que l'un quelconque parmi R² à R⁵ à sa position 3, 4 ou 6 ;
A est choisi dans le groupe constitué de : -N = N- ; -S-S- ; -As = As- ; une chaîne linéaire ayant de 1 à 3 atomes de carbone et ayant une simple, double ou triple liaison carbone-carbone facultativement conjointement avec un substituant oxo (=O) sur l'un des atomes de carbone dans la chaîne ; -C=C- ; un alkyle de 1 à 6 carbones non substitué ou à substitution trans ou cis -CH = CH-, -CH₂-CH₂-, -CO-CH = CH-, -CH = CH-CO-, -CO-, -CH₂-CO-, ou -CH₂ ;
R est choisi parmi l'hydrogène ou un alkyle de 1 à 6 carbones facultativement substitué avec des substituants choisis parmi R² à R⁵ ;
R¹ est -OH ou -NH₂, et facultativement substitué par un alkyle de 1 à 6 carbones facultativement substitué avec l'un quelconque parmi R² à R⁵;
R² est l'hydrogène, ou un acyle de 1 à 7 carbones facultativement substitué avec l'un quelconque parmi R² à R⁵ ; et
R³, R⁴, R⁵ sont indépendamment l'hydrogène, un alkyle de 1 à 6 carbones, un halogène, l'hydroxy, le cyano, le carboxy, un alcoxy de 1 à 6 carbones, le benzyloxy, un acyle de 1 à 7 carbones, dans lequel l'un quelconque des noyaux benzéniques est facultativement substitué avec un alkyle de 1 à 6 carbones.

2. Utilisation de la revendication 1, dans laquelle Ph¹ est le benzène.

3. Utilisation de la revendication 1, dans laquelle Ph² est C₆H₂R⁶ et R6 est l'hydrogène, l'hydroxy, un halogène ou un alkyle de 1 à 6 carbones.

4. Utilisation de la revendication 1, dans laquelle A est choisi parmi -N=N-, -C≡C- ; -CH = CH- ; -CH₂-CH₂ ; -CO-CH=CH- ; -CH=CHCO- ; ou -CH₂-CO-.

5. Utilisation de l'une quelconque des revendications 1 à 4, dans laquelle le cancer est *cys*^{*-*}*.*

6. Utilisation de l'une quelconque des revendications 1 à 5, dans laquelle l'acide ou amide salicylique est couplé à la position de cycle par un groupe de pontage qui est stable contre l'hydrolyse ou la réduction dans des systèmes biologiques.

7. Utilisation de l'une quelconque des revendications 1 à 6, dans laquelle le composé est la sulfasalazine, la disalazine ou la salazosulfadimidine.

8. Utilisation de la revendication 7, dans laquelle le composé est la sulfasalazine.

9. Utilisation de la revendication 7 ou 8, dans laquelle le composé est pour administration intrapéritonéale ou intraveineuse.

10. utilisation de l'une quelconque des revendications 1 à 9, dans laquelle le composé est incorporé dans un liposome.

11. Composition pharmaceutique comprenant le composé de la revendication 1 incorporé dans un liposome.

12. Composition de la revendication 11, dans laquelle le composé est un composé des revendications 2, 3 ou 4.

13. Composition de la revendication 12, dans laquelle le composé est la sulfasalazine, la disalazine ou la salazosulfadimidine.

14. Composition de la revendication 13, dans laquelle le composé est la sulfasalazine.
